# EUROPEAN PATENT APPLICATION

(11) **EP 3 072 956 A1**
(43) Date of publication of application: **28.09.2016**
(21) Application number: 15160765.2
(22) Date of filing: 25.03.2015
(51) Int. Cl.: C12N 1/20, C12R 1/01

(54) **Selective culture medium for polymyxin-resistant, gram-negative bacteria**

(71) Applicant: Université de Fribourg, 1700 Fribourg (CH)
(72) Inventor: Nordmann, Patrice, 1700 Fribourg (CH); Poirel, Laurent, 1700 Fribourg (CH); Jayol, Aurélie, 1700 Fribourg (CH)
(74) Representative: Schneiter, Sorin

(57) **Abstract**

The present invention concerns culture medium for specifically selecting gram-negative, aerobe bacteria that have an acquired resistance to polymyxins as well as bacteria that are naturally resistant to polymyxins. In particular, the culture medium allows selection of *Enterobacteriaceae, Pseudomonas aeruginosa* and *Acinetobacter baumannii* having an acquired resistance to polymyxins. Presently existing culture media select naturally resistant bacteria, but were found not to select bacteria that have an acquired resistance to polymyxins. The use of polymyxin antibiotics in humans has increased recently, as a last resort treatment against infections by multidrug resistant (MDR) bacteria. The present invention identifies and resolves the need for a diagnostic test for the identification and characterization of bacteria that have an acquired resistance to polymyxins as well.

## Description

### Technical Field

The present invention relates to a culture medium and to a method for specifically selecting gram-negative, aerobic bacteria that have an acquired resistance to polymyxins as well as bacteria that are naturally resistant to polymyxins.

### Background Art and Problems Solved by the Invention

Multidrug resistance (MDR) to antibiotics in bacteria is becoming a major issue worldwide. Those bacteria account for most hospital infections worldwide, affecting, for example in the USA, two million people and causing 25,000 deaths annually. Many important public bodies have already raised an alarm signal on the rapid and uncontrollable spread of MDR bacteria such as the WHO, the European Center for Control Diseases in Stockholm and the Center for Diseases control (CDC) in Atlanta (USA), and most recently the International Monetary Fund and the White House (USA).

The most important sources of concern are MDR Gram negative bacteria such as *Escherichia coli* and *Klebsiella pnearmoniae, Pseudomonas aeruginosa* and *Acinetobacter baumannii* since those bacterial species are the main sources of infections in humans, with respect to both, community- and hospital-acquired infections. Those infections are mostly urinary tract infections, bloodstream infections, pulmonary and intra-abdominal infections. Their treatment mostly relies on ß-lactams such as penicillins, cephalosporins, the broadest spectrum ß-lactams i.e. carbapenems, aminoglycosides and quinolones. Acquired resistance to those antibiotic molecules is increasingly reported worldwide, being often associated in MDR bacteria. Very few, if any, therapeutic options are left. Therefore, a renewed interest in an old class of antibiotics, polymyxins, colistin and polymyxin B, is observed worldwide. As a recent example, large use of polymyxins is now observed for treating bloodstream infections due to *Klebsiella pneumoniae* in Italy which shows a very high prevalence rate (40-60%) of carbapenem resistance due to the spread of carbapenemase producers. The large use of polymyxins in this country explains why colistin resistance superimposes to carbapenem resistance in *Enterobacteriaceae.*

The polymyxins became clinically available in the 1950's but soon fell out of favor mainly because of concerns of their potential toxic effects for kidneys and development of many efficient and much less toxic antibiotics, in particular cephalosporins, carbapenems, aminoglycosides, and fluoroquinolones. Polymyxin molecules were mostly used in veterinary medicine. They have been used in human medicine as an attempt to decolonize patients carrying MDR bacteria.

Being products of bacterial fermentation (from *Bacillus polymyxa* subspecies *colistinus*), colistin and polymyxin B are multicomponent antibiotics active only against several gram-negative aerobes. They have very similar chemical structures, differing by one amino acid in the peptide ring. The polymyxins are relatively large lipopeptide molecules and are poorly absorbed after oral administration. For the treatment of life-threatening systemic infections, they are given by the intravenous route or by nebulization for the treatment of respiratory tract infections. Likewise other antimicrobial peptides, colistin interacts with the lipid A moiety of the lipopolysaccharide (LPS) of gram-negatives. The polycationic peptide ring competes for and substitutes the calcium and magnesium bridges stabilizing the LPS, promoting membrane permeability and thus disrupting the integrity of the outer membrane of gram negative, thus leading to bacterial death.

Natural polymyxin resistance is known in several gram negatives species such as in *Proteus sp, Serratia sp* and *Burkholderia sp.* Acquired resistance to polymyxins is increasingly observed and this is the most important source of concern in the polymyxin resistance domain. Although polymyxin resistance rates remain relatively low in developed countries, except in Italy, Spain and Greece, there is a concern that this situation might change rapidly. The main mechanisms of acquired resistance to polymyxins in *Enterobacteriaceae,* in particular in *K. pneumoniae*, are modifications in genes modifying the LPS leading to a weak, if any, binding of polymyxins to the outer-membrane proteins.

No medium is available for screening bacteria with acquired resistance to polymyxins that account for variable levels of resistance to polymyxins. Therefore, a selective culture media for screening those polymyxin-resistant strains is needed.

Such a medium will be also needed in veterinary medicine to screen for polymyxin-resistant isolates since polymyxins are widely used for prophylaxis, metaphylaxis and treatment of animals, for example, for the treatment of diarrhea in cows.

Polymyxins have been used as selective agent as a component of several selective culture media to eliminate polymyxin-susceptible gram negatives from contaminated flora. One of those examples of the use of polymyxin B is the *Bacillus anthracis* medium. Media containing polymyxins are available for screening bacterial strains that are naturally resistant to polymyxins, such as *Burkholderia cepacia* and *Serratia marcescens.*

The present invention seeks to address the problems related to multidrug resistance by providing a rapid and innovative test for identifying and characterizing antibiotic-resistant bacteria, in particular bacteria that exhibit an acquired resistance to polymyxins.

### Summary of the Invention

Remarkably, the inventors propose a method and a medium for detecting bacteria displaying a natural as well as acquired phenotype of resistance to polymyxins.

In an aspect, the present invention provides a culture medium comprising a polymyxin antibiotic, a daptomycin antibiotic and an antifungal agent.

In another aspect, the invention provides method for selecting gram-negative, aerobic bacteria that have an acquired resistance to polymyxins as well as bacteria that are naturally resistant to polymyxins. The method comprising the steps of: providing the culture medium of the present invention; inoculating said culture medium with a sample comprising bacteria; incubating said inoculated culture medium under conditions suitable for the growth of bacteria, in particular bacteria exhibiting natural or acquired resistance to polymyxins; detecting colonies formed on said inoculated and cultivated medium, wherein said colonies containing said bacteria that exhibit natural and/or acquired resistance to polymyxins.

In an aspect, the present invention provides a method for detecting bacteria that have an acquired resistance to polymyxins as well as bacteria that are naturally resistant to polymyxins in a test sample, the method comprising the steps of: providing the culture medium of the present invention; inoculating said culture medium with said test sample; incubating said inoculated culture medium under conditions suitable for the growth of bacteria, in particular of bacteria exhibiting natural or acquired resistance to polymyxins; detecting colonies formed on said inoculated and cultivated medium, said colonies containing said bacteria that exhibit natural and/or acquired resistance to polymyxins.

Further aspects and preferred embodiments are defined in the appended claims and the detailed description herein below.

### Detailed Description of the Preferred Embodiments

In an aspect, the present invention provides a culture medium comprising a polymyxin antibiotic, a daptomycin antibiotic and an antifungal agent.

The term "comprising", for the purpose of the present specification, is intended to mean "comprises, amongst other". It is not intended to mean "consists only of".

The culture medium of the invention is preferably a gel or liquid designed to support the growth of microorganisms, in particular the growth of gram-negative bacteria. The culture medium may be a nutrient broth or an agar plate, for example. Preferably the culture medium is solid, that is, gel-based. Preferably, the culture medium is agar-based. The culture medium of the invention is preferably a selective medium, supporting the growth of selected microorganisms only. In an embodiment, the culture medium is a selective as well as an indicator medium, which allows identifying characteristics of the microorganism growing on the medium.

The culture medium preferably comprises nutrients for supporting the growth of the microorganisms that are to be selected. The culture medium preferably comprises a carbon source, in particular carbohydrates, such as poly-, oligo-, di- and/or monosaccharides that can be metabolized by the bacteria to be selected. Preferably, the culture medium comprises di- and/or monosaccharides. In an embodiment, the culture medium may contain one or more selected from sucrose, glucose and lactose. In a preferred embodiment, the culture medium contains lactose and sucrose as carbon sources supporting the growth of microorganisms. As a source of amino acids, the culture medium may contain peptone, for example. The culture medium preferably comprises various salts needed for bacterial growth.

In a preferred embodiment, the culture medium of the invention is based on and/or comprises Eosine Methylene Blue (EMB) Culture Medium, according to the formulation described by M. Levine in 1918 (J Infect Dis 23: 43-47). The EMB medium is a selective-differential plating medium for the detection and isolation of gram-negative bacteria.

The present invention seeks to provide a culture medium that allows for the rapid detection of bacteria that have an acquired resistance as well as bacteria that have a natural resistance to polymyxins antibiotics. Therefore, the culture medium comprises a polymyxin antibiotic.

In an embodiment, the polymyxin antibiotic is selected from colistin, polymyxin B and mixtures thereof.

In an embodiment, the culture medium comprises only one polymyxin antibiotic, which is colistin. Preferably, colistin is colistin according to CAS number 1264-72-8.

In another embodiment, the culture medium comprises only one polymyxin antibiotic, which is polymyxin B. Preferably, polymyxin B is the molecule according to CAS number 1405-20-5.

In an embodiment, the said polymyxin antibiotic is present at a concentration of 0.01 to 6 µg/ml in said culture medium. Preferably, this concentration applies to the total concentration of all polymyxins present in the culture medium, in case a mixture of different polymyxin antibiotics, such as a mixture of colistin and polymyxin B is present in the culture medium.

In a preferred embodiment, said polymyxin antibiotic is present at a concentration of 0.4 to 5 µg/ml, preferably 1 to 4 µg/ml, more preferably from > 2 to < 4 µg/ml.

In a most preferred embodiment, said polymyxin antibiotic is present at a concentration of 3.0 to 4 µg/ml, for example 3 to 3.6 µg/ml.

The inventors found that for identifying bacteria that have resistance to polymyxin antibiotics, concentrations more than 2 µg/ml are preferred. This is surprising, given that the EUCAST (European Committee in Antimicrobial Susceptibility Testing) has determined the minimum inhibitory concentration (MIC) values of polymyxins to be ≤ 2 mg/L and resistance >2 mg/L for *Enterobacteriaceae* and *Acinetobacter baumannii*, and susceptibility ≤ 4 mg/L and resistance >4 mg/L for *Pseudomonas aeruginosa*. The present inventors have identified a polymyxin concentration that is suitable to select for most if not all types of resistances, including acquired resistances, which are generally not susceptible but not resistant to polymyxins, either.

The medium of the present invention is suitable to select for bacteria that are "naturally resistant" to polymyxins. This group of bacteria contains bacteria that have originally never been susceptible to polymyxins, in particular in the taxa of *Proteus sp, Serratia sp* and *Burkholderia sp.*

On the other hand, the medium of the present invention is intended to select for, detect and/or identify bacteria that have an acquired resistance to polymyxins. This group of bacteria has developed a resistance or a partial resistance upon exposure to the antibiotic and is thus different from the naturally resistant group. Acquired resistance is observed in other bacterial taxa, in particular in *Enterobacteriaceae,* such as *Escherichia coli* and *Klebsiella pneumoniae*; *Acinetobacter baumannii*, and *Pseudomonas aeruginosa*. Table 1 below provides a list containing bacterial taxa that are reported to have acquired partial or total resistance.

The present inventors observed that bacteria having an acquired resistance to polymyxins are generally not as resistant to polymyxins as the naturally resistant bacteria. Bacteria having an acquired resistance to polymyxins are generally partially resistant and/or partially susceptible to polymyxins. Furthermore, the resistance and/or susceptibility of bacteria having an acquired resistance are highly variable and based on various different molecular mechanisms. For this reason it is surprising to provide a selective medium containing polymyxins at a determined concentration or concentration range that is suitable to select for all bacteria having an acquired resistance to polymyxins, or at least all bacteria that were tested in the experiments conducted by the inventors.

In a preferred embodiment, the culture medium of the invention comprises a daptomycin antibiotic. The daptomycin antibiotic is preferably selected from daptomycin and analogues thereof, preferably it is daptomycin. In a preferred embodiment, daptomycin is daptomycin having CAS number 103060-53-3.

The present inventors observed that, if an EMB culture medium is used, the growth of gram-positive bacteria is not sufficiently inhibited by the dye methylene blue and eosin. Therefore, the inventors determined that a further antibiotic, specific to gram-positive bacteria, is preferably present. The present inventors have tested several antibiotics to this end, but not all of the tested antibiotics fulfilled the purpose in accordance with the present invention. Surprisingly, excellent results were achieved when using daptomycin, in particular in connection with said EMB medium.

In an embodiment, the concentration of said daptomycin antibiotic in the culture medium of the invention is 0.5 to 50 µg/ml, preferably 1 to 30 µg/ml, more preferably 5 to 15 µg/ml. A concentration of 8 to 12 µg/ml is most preferred.

The culture medium of the present invention preferably comprises an antifungal agent. Preferably, the antifungal agent is amphotericin B.

In an embodiment, the concentration of said antifungal agent, in particular amphotericin B in the culture medium of the invention is 0.1 to 50 µg/ml, preferably 0.5 to 25 µg/ml, more preferably 1 to 10 µg/ml, and most preferably 3-7 µg/ml.

In an embodiment, the culture medium of the invention is EMB agar which contains peptone, lactose, sucrose, and the dyes eosin and methylene blue. It is commonly used as both a selective medium for Gram-negative bacteria and a differential medium.

As mentioned above, the combination of eosin and methylene blue dyes inhibits most gram-positive bacteria and allows the selection of the gram-negative bacteria. The eosin dye allows also the differentiation between lactose fermenters and lactose non fermenters. It responds to decrease in pH when lactose is fermented, going from colorless to black. Therefore, lactose fermenters as *E. coli* and *E. cloacae* possess dark centers when cultured on EMB a medium containing eosin and methylene.

On the other hand, sucrose included in an eosin and methylene blue-containing medium allows the differentiation between coliforms that are able to ferment sucrose more rapidly than lactose and those that are unable to ferment sucrose. The vigorous lactose and sucrose fermentation of *E. coli* on a medium containing lactose and sucrose as a carbon sources causes a bigger amount of acid production than for only lactose-fermenting bacteria and is associated with a green metallic sheen. Therefore, *E. coli* bacteria will give a distinctive green-metallic sheen with a dark center. Lactose or sucrose non-fermenters as *Proteus mirabilis* are colorless and appeared therefore translucent or pink

In an embodiment, the culture medium comprises chromogenic components, in particular chromogenic components other than and/or in addition to eosine and methylene blue. Such chromogenic components may allow the rapid recognition of bacterial taxa. In a preferred embodiment, the culture medium comprises chromogenic compounds for specifically detecting polymyxin resistant *Acinetobacter baumannii* and *Pseudomonas aeruginosa*. For example, the presence of ß-alanylarylamidase activity in certain bacteria, such as *Pseudomonas*, can be determined using 7-Amido-1-pentyl-phenoxazin-3-one or other chromogenic compounds in the culture medium.

In a preferred embodiment, the present invention provides an EMB agar-based culture medium comprising a polymyxin selected from colistin and polymyxin B, daptomycin and amphotericin B. In a preferred embodiment, this culture medium comprises 1-5 µg/ml of said polymyxin; 2 to 20 µg/ml of daptomycin and 1-10µg/ml of amphotericin B.

In a more preferred embodiment, the invention provides an EMB agar gel-based culture medium comprising more than 2 and less of 4 µg/ml of said polymyxin, 5 to 15 µg/ml of daptomycin and 3-9 µg/ml of amphotericin B.

In a most preferred embodiment, the invention provides an EMB agar gel-based culture medium comprising 3 to 3.6 µg/ml of said polymyxin, 5 to 15 µg/ml of daptomycin and 3-9 µg/ml of amphotericin B.

The present invention provides a method for identifying bacteria having an acquired or a natural resistance to polymyxins and a method for selecting and/or detecting such bacteria.

The methods of the invention preferably comprise the steps of:
- providing a culture medium of the present invention;
- inoculating said culture medium with a test sample;
- incubating said inoculated culture medium under conditions suitable for the growth of bacteria, in particular for the growth of bacteria exhibiting natural or acquired resistance to polymyxins;
- detecting colonies formed on said inoculated and cultivated medium, said colonies containing said bacteria that exhibit natural and/or acquired resistance to polymyxins.

For the purpose of the present specification, a "test sample" means any liquid or solid material to be tested, which may contain bacteria that have an acquired or natural resistance to polymyxins. Preferably, the "test sample" is a biological sample.

For the purpose of the present specification, a "biological sample" means any biological sample derived from a subject. Examples of such samples include body fluids, skin swabs, tissues, cell samples, etc. Preferred biological samples are saliva, whole blood, serum, plasma, urine or faeces.

In an embodiment, the method of the invention can be applied directly to a raw test sample, such as a raw biological sample, comprising a mixture of bacteria, without having to isolate or separate the various bacterial strains present in the sample.

The culture medium and the methods of the invention have the advantage of eliminating polymyxin-susceptible gram negatives, gram-positives and fungi that are usually part of many bacterial floras.

As used herein, a "subject" denotes a human or animal subject, wherein said animal subject is preferably a mammal. The animal subject may be selected from artyodacyla, in particular land-living, domesticated artyodactyla, such as cattle and pigs, perissodactyla, such as horses, carnivores, such as dogs and cats, rodents and primates.

In a preferred embodiment, the "subject" is a human or a domestic animal, in particular a human or a livestock animal or companion animals

The methods of the invention may be used to detect bacteria that exhibit at least a partial resistance to polymyxins, such as bacteria having a reduced susceptibility, which means a decreased susceptibility compared to wild-type polymyxin susceptibility.

In an embodiment of the methods and the culture medium of the invention, said bacteria that have an acquired resistance to polymyxins are bacteria belonging to taxa selected from the group of *Enterobacteriaceae, Pseudomonas aeruginosa* and *Acinetobacter baumannii*.

In an embodiment of the methods and the culture medium of the invention, said bacteria that have a natural resistance to polymyxins are bacteria belonging to taxa selected from the group of *Burkholderia*, *Proteus* and *Serratia.*

In an embodiment of the methods of the invention, said step of incubating said inoculated culture medium under conditions suitable for the growth of bacteria exhibiting natural or acquired resistance to polymyxins (said incubation step), is conducted for 24 hours to 48 hours, preferably 24 to 36 hours.

Conditions suitable for the growth bacteria exhibiting natural or acquired resistance to polymyxins can be determined by the skilled person. Typically, such conditions include a temperature of about 30-39°C, preferably 35-37°C, the presence of water, salts, and macro and micronutrients required for bacterial growth. Such nutrients are contained, for example, in the EMB agar as disclosed elsewhere in this specification. Other media are likewise suitable to provide nutrients suitable for bacterial growth in accordance with the invention.

In an embodiment of the methods of the invention, the detection of bacteria that have an acquired resistance to polymyxins is determined by detecting the occurrence of colonies within a culturing time of 24 to 36 hours. Bacteria that are naturally resistant to polymyxins, such as *Burkholderia cepacia* and *Stenotrophomonas maltophilia,* are also detected within this time frame.

The step of detecting colonies formed on said inoculated and cultivated medium is preferably accomplished by visual inspection. In particular, the presence of colonies is determined visually by a trained person capable of identifying bacterial colonies. The presence of such colonies indicates the presence of growth of bacteria, wherein growth generally means exponential growth. Accordingly, the presence of a colony on the culture medium indicates that said bacteria are in an exponential growth phase within the above specified time frames of 24 hours to 48 hours, preferably 24 to 36 hours.

In an embodiment, the detection of naturally resistant *Burkholderia cepacia* and *Stenotrophomonas maltophilia* is determined by detecting the occurrence of colonies at a culturing time of 36 hours or more. Surprisingly, these naturally resistant bacteria generally enter an exponential growth only at or after 36 hours on the culture medium of the present invention.

### Examples

### Material and Methods

*Antimicrobial agents, reagents, and materials.* Standard colistin sulfate and polymyxins B were from Sigma Aldrich (Saint-Louis, USA). Those antibiotic powders were kept at 4°C before their use and polymyxin dilutions were kept at -20°C. They were used for MIC values determination and for determining the optimal polymyxin concentration to be used in the SUPERPOLYMYXIN medium. Daptomycin and amphotericin B were from Novartis (Horsham, UK) and Bristol-Myers-Squibb (Reuil-Malmaison, France), respectively. Tween 80 was from Sigma, cation-adjusted Mueller-Hinton powder from bioRad (Marnes-La-Coquette, France) were used for MIC determinations.

*Bacterial strains.* Sixty-two enterobacterial strains were studied (Table 1). Thirty-nine enterobacterial strains were resistant to polymyxins. They were from worldwide origin (Europe, America, Africa) and from human and veterinary medicine. In most of the cases, resistance to colistin/polymyxin B was associated to gene mutation or gene disruption in genes modifying the LPS structure (Table 1, part II). In addition, four polymyxin-resistant non-fermenters (*Burkholderia cepacia, Stenotrophomonas maltophilia, Acinetobacter baumannii)* and seven polymyxin-susceptible non fermenters were studied (A. *baumannii*, *P. aeruginosa)*.

*Determination of MIC values* were performed by broth microdilution in Cation-Adjusted Mueller-Hinton broth (MHB-CA) according to the CLSI guidelines (Clinical and Laboratory Standards Institute). Polymyxin B and colistin (Sigma-Aldrich, Saint-Louis) were tested with or without Tween 80 over a range dilution from 0.12 to 128 µg/ml. The surfactant Tween 80, at a final concentration of 0.002%, prevents the binding of polymyxins to multi-array plastic panel. Actually, the use of Tween for determination of MIC of polymyxins is still debatable. MHB-CA broth and 5.10⁵ CFU/ml of each tested strain were distributed in each well. Following the EUCAST breakpoints, strains were characterized as susceptible or resistant to polymyxins.

*Screening for polymyxin resistance by using the SUPERPOLYMYXIN medium.* This medium contains Eosine Methylene Blue (agar) (EMB, Fluka) as described by Levine in 1918. It is able to select for gram negatives distinguishing between lactose-positive and lactose-negative bacteria. However, several strepotococci and staphylococci may still grow as small colony variants. *Escherichia coli* grow with a green metallic color with a dark center whereas *Klebsiella pneumoniae* grows with a brownish color with dark-centered mucoid colonies. Several colistin and polymyxin B concentrations were studied and colistin at a concentration of 3.5 mg per L was retained to be added to select for colistin resistant strains. Daptomycin (10 mg/L) was added since the EMB medium by itself did not inhibit the growth of all gram-positive bacteria (see above), and amphotericin B to inhibit yeasts and fungi growth (5 mg/L). Using an inoculum of ~1 x 10⁸ CFU/ml (0.5 Mac Farland), serial 10-fold dilutions of the isolates were made in NaCl 0.85%and were plated onto the SUPERPOLYMYXIN medium. Viable bacteria were counted after 24 hours of culture (and 36 h for *B. cepacia* and *S. maltophilia*) at 37°C and growth on selective SUPERPOLYMYXIN medium was compared to growth on EMB agar without colistin. The polymyxin-susceptible and -resistant strains reported in Table 1 (part I) were tested. The lowest limit of detection of the tested stains were determined using the SUPERPOLYMYXIN medium. The sensitivity and specificity cutoff values were set at 1 x 10³ CFU/ml, which means a limit value of 1 x 10³ CFU/ml and above was considered « not efficiently detected ».

**Table 1 (part I): Bacterial strains tested and determination of MICs of polymyxins**

| Strain | Species | Origin of the strains | MIC colistin (mg/l)^{a} | | MIC polymyxin B (mg/l)^{a} | |
|---|---|---|---|---|---|---|
| | | | Without Tween80 | With Tween80 | Without Tween80 | With Tween80 |
| Natural colistin-resistant strains | | | | | | |
| Pmir | *P. mirabilis* | France | >128 | >128 | >128 | >128 |
| Pvul | *P. vulgaris* | France | >128 | >128 | >128 | >128 |
| Pstu | *P. stuartii* | France | >128 | >128 | >128 | >128 |
| Mmor | *M. morganii* | France | >128 | >128 | >128 | >128 |
| Smar | *S. marcescens* | France | >128 | >128 | >128 | >128 |
| Bcep | *B. cepacia* | France | >128 | >128 | >128 | >128 |

| Strains showing an acquired resistance trait to colistin | | | | | | |
|---|---|---|---|---|---|---|
| C25 | *K. pneumoniae* | Colombia | 64 | 32 | 64 | 32 |
| AF1b | *K. pneumoniae* | South Africa | 16 | 16 | 8 | 8 |
| C3 | *K. pneumoniae* | Colombia | 32 | 32 | 32 | 16 |
| 75b | *K. pneumoniae* | South Africa | 128 | 128 | 128 | 128 |
| 1515 | *K. pneumoniae* | France | 64 | 32 | 32 | 32 |
| Sa | *K. pneumoniae* | France | 128 | >128 | 64 | 64 |
| C11 | *K. pneumoniae* | Colombia | 64 | 128 | 128 | 64 |
| 12E2 | *K. pneumoniae* | France | 64 | 64 | 64 | 64 |
| C1 | *K. pneumoniae* | Colombia | 128 | 128 | 128 | 64 |
| T5 | *K. pneumoniae* | Turkey | 32 | 64 | 32 | 32 |
| BICb | *K. pneumoniae* | France | >128 | >128 | 128 | 128 |
| T1b | *K. pneumoniae* | Turkey | 128 | 128 | 64 | 64 |
| 1118 | *K. pneumoniae* | France | 64 | 64 | 32 | 32 |
| C21 | *K. pneumoniae* | Colombia | 128 | 128 | 128 | 128 |
| C22 | *K. pneumoniae* | Colombia | 32 | 64 | 64 | 64 |
| C26 | *K. pneumoniae* | Colombia | 128 | 128 | 128 | 128 |
| T4 | *K. pneumoniae* | Turkey | 64 | 64 | 32 | 32 |
| C27 | *K. pneumoniae* | Colombia | >128 | >128 | >128 | >128 |
| C12 | *K. pneumoniae* | Colombia | 64 | 64 | 128 | 64 |
| C24 | *K. oxytoca* | Colombia | 64 | 64 | 64 | 64 |
| 13G2 | *K. pneumoniae* | France | 64 | 32 | 64 | 64 |
| 18J1 | *K. pneumoniae* | France | 32 | 32 | 32 | 32 |
| C7 | *K. pneumoniae* | France | >128 | >128 | >128 | >128 |
| C16 | *K. pneumoniae* | France | 64 | 64 | 32 | 64 |
| 28509 | *E. coli* | France | 4 | 4 | 2 | 2 |
| 30742 | *E. coli* | France | 8 | 4 | 4 | 4 |
| 27563 | *E. coli* | France | 16 | 8 | 16 | 8 |
| 27837 | *E. coli* | France | 16 | 8 | 8 | 8 |
| 27850 | *E. coli* | France | 16 | 8 | 8 | 8 |
| 27852 | *E. coli* | France | 8 | 8 | 4 | 4 |
| C28 | *E. cloacae* | Colombia | 32 | 32 | 16 | 16 |
| HM | *E. cloacae* | France | >128 | >128 | >128 | >128 |
| ER | *H. alvei* | France | 16 | 16 | 8 | 16 |
| Aba6 | *A. baumannii* | Switzerland | 128 | 128 | 128 | 128 |
| Sm2 | *S. maltophilia* | France | >128 | >128 | >128 | >128 |
| SmA51 | *S. maltophilia* | France | 32 | 16 | 32 | 32 |

| Strains susceptible to colistin | | | | | | |
|---|---|---|---|---|---|---|
| ATCC 25922 | *E. coli* | Reference strain | 0.25 | 0.12 | 0.25 | 0.12 |
| ATCC 27853 | *P. aeruginosa* | Reference strain | 0.5 | 0.12 | 0.5 | 0.12 |
| Eco OmpC-OmpF- | *E. coli* | Reference strain | 0.12 | 0.12 | 0.125 | 0.12 |
| Kp OmpK3 5-OmpK3 6- | *K. pneumoniae* | Reference strain | 0.12 | 0.12 | 0.125 | 0.12 |
| 36A4 | *K. pneumoniae* | France | 0.12 | 0.12 | 0.25 | 0.25 |
| 36A5 | *K. pneumoniae* | France | 0.12 | 0.12 | 0.12 | 0.12 |
| 36A6 | *E. coli* | France | 0.12 | 0.12 | 0.12 | 0.12 |
| 36A7 | *K. pneumoniae* | France | 0.25 | 0.12 | 0.5 | 0.5 |
| 36D7 | *E. coli* | France | 0.25 | 0.12 | 0.12 | 0.12 |
| 36F8 | *E. aerogenes* | France | 0.12 | 0.12 | 0.12 | 0.12 |
| 38A10 | *E. cloacae* | France | 0.12 | 0.12 | 0.25 | 0.25 |
| 38B2 | *C. freundii* | France | 0.12 | 0.12 | 0.5 | 0.5 |
| 38B7 | *K. pneumoniae* | France | 0.5 | 0.12 | 0.12 | 0.5 |
| 38B9 | *A. baumannii* | France | 0.5 | 0.25 | 0.5 | 0.25 |
| 38D6 | *E. coli* | France | 0.25 | 0.12 | 0.12 | 0.12 |
| 38J8 | *P. aeruginosa* | France | 2 | 0.5 | 1 | 0.25 |
| C105 | *A. baumannii* | Colombia | 0.5 | 0.12 | 0.5 | 0.12 |
| C111 | *P. aeruginosa* | Colombia | 1 | 0.12 | 1 | 0.25 |
| C112 | *K. pneumoniae* | Colombia | 0.5 | 0.12 | 0.25 | 0.25 |
| C115 | *K. pneumoniae* | Colombia | 0.12 | 0.12 | 0.5 | 0.12 |
| C129 | *E. cloacae* | Colombia | 0.12 | 0.12 | 0.25 | 0.25 |
| C130 | *K. pneumoniae* | Colombia | 0.5 | 0.12 | 0.25 | 0.5 |
| C131 | *P. aeruginosa* | Colombia | 1 | 0.25 | 1 | 0.25 |
| C132 | *K. pneumonia* | Colombia | 0.12 | 0.12 | 0.25 | 0.5 |
| C133 | *P. aeruginosa* | Colombia | 2 | 0.5 | 1 | 0.5 |
| C136 | *C. freundii* | Colombia | 0.25 | 0.12 | 0.5 | 0.25 |
| C140 | *E. coli* | Colombia | 0.12 | 0.12 | 0.25 | 0.12 |
| C144 | *E. cloacae* | Colombia | 0.12 | 0.12 | 0.25 | 0.12 |

| | | | | | | |
|---|---|---|---|---|---|---|
| ^{a} MICs of colistin and polymyxin were determined by broth microdilution with and without Tween 80 at the final concentration of 0.002 %. | | | | | | |

**Table 1 (part II): Limits of detection of the SUPERPOLYMYXIN medium**

| Strain | Species | Polymyxin resistance | Resistance mechanism to polymxyins | Lowest detection limit (CFU/mL) ^{b} |
|---|---|---|---|---|
| Natural colistin-resistant strains | | | | |
| Pmir | *P. mirabilis* | + | Natural resistance | 10¹ |
| Pvul | *P. vulgaris* | + | Natural resistance | 10¹ |
| Pstu | *P. stuartii* | + | Natural resistance | 10¹ |
| Mmor | *M. morganii* | + | Natural resistance | 10¹ |
| Smar | *S. marcescens* | + | Natural resistance | 10¹ |
| Bcep | *B. cepacia* | + | Natural resistance | 10^{1 c} |

| Strains showing an acquired resistance trait to colistin | | | | |
|---|---|---|---|---|
| C25 | *K. pneumoniae* | + | PmrA G53C | 10¹ |
| AF1b | *K. pneumoniae* | + | PmrB T157P | 10¹ |
| C3 | *K. pneumoniae* | + | PmrB T157P | 10¹ |
| 75b | *K. pneumoniae* | + | PhoP D191Y | 10¹ |
| 1515 | *K. pneumoniae* | + | MgrB truncated with codon stop | 10¹ |
| Sa | *K. pneumoniae* | + | MgrB truncated with codon stop | 10¹ |
| C11 | *K. pneumoniae* | + | MgrB truncated with codon stop | 10¹ |
| 12E2 | *K. pneumoniae* | + | MgrB N42Y and K43I | 10¹ |
| C1 | *K. pneumoniae* | + | *mgrB* promotor truncated with IS10R (-26 and -27) | 10¹ |
| T5 | *K. pneumoniae* | + | *mgrB* promotor truncated with ISKpn14 (-27 and -28) | 10¹ |
| BICb | *K.pneumoniae* | + | *mgrB* gene truncated with IS-102 like (+44 and +45) | 10¹ |
| T1b | *K. pneumoniae* | + | *mgrB* gene truncated with IS5-like (+74 and +75) | 10¹ |
| 1118 | *K. pneumoniae* | + | *mgrB* gene truncated with IS5-like (+74 and +75) | 10¹ |
| C21 | *K. pneumoniae* | + | *mgrB* gene truncated with ISKpn13 (+74 and +75) | 10¹ |
| C22 | *K. pneumoniae* | + | *mgrB* gene truncated with ISKpn14 (+127 and +128) | 10¹ |
| C26 | *K. pneumoniae* | + | *mgrB* gene truncated with IS (+ and +) | 10¹ |
| T4 | *K. pneumoniae* | + | *mgrB* gene truncated with IS903b-like (+69 and +70) | 10¹ |
| C27 | *K. pneumoniae* | + | *mgrB* Δnt23/33 (frameshift) | 10¹ |
| C12 | *K. pneumoniae* | + | Δ*mgrB* | 10¹ |
| C24 | *K. oxytoca* | + | *mgrB* promotor truncated with ISKpn26-like (-38 and -39) | 10¹ |
| 13G2 | *K. pneumoniae* | + | Unknown | 10¹ |
| 18J1 | *K. pneumoniae* | + | Unknown | 10¹ |
| C7 | *K. pneumoniae* | + | Unknown | 10¹ |
| C16 | *K. pneumoniae* | + | Unknown | 10¹ |
| 28509 | *E. cloacae* | + | Unknown | 10² |
| 30742 | *E. cloacae* | + | Unknown | 10¹ |
| 27563 | *E. cloacae* | + | Unknown | 10¹ |
| 27837 | *E. cloacae* | + | Unknown | 10¹ |
| 27850 | *E. cloacae* | + | Unknown | 10¹ |
| 27852 | *E. cloacae* | + | Unknown | 10¹ |
| C28 | *E. cloacae* | + | Unknown | 10¹ |
| HM | *E. cloacae* | + | Unknown | 10¹ |
| ER | *H. alvei* | + | Unknown | 10¹ |
| Aba6 | *A. baumannii* | + | Unknown | 10² |
| Sm2 | *S. marcescens* | + | Unknown | 10¹ |
| SmA51 | *S. marcescens* | + | Unknown | 5x10^{2 c} |
| Strains susceptible to colistin | | | | |
| ATCC 25922 | *E. cloacae* | - | NA | >10⁷ |
| ATCC 27853 | *P. aeruginosa* | | NA | 5x10⁶ |
| Eco OmpC-OmpF- | *E. cloacae* | - | NA | >10⁷ |
| Kp OmpK35-OmpK36- | *K. pneumoniae* | - | NA | 5x10⁶ |
| 36A4 | *K. pneumoniae* | - | NA | >10⁷ |
| 36A5 | *K. pneumoniae* | - | NA | >10⁶ |
| 36A6 | *E. cloacae* | - | NA | >10⁷ |
| 36A7 | *K. pneumoniae* | - | NA | >10⁶ |
| 36D7 | *E. cloacae* | - | NA | >10⁷ |
| 36F8 | *E. aerogenes* | - | NA | >10⁷ |
| 38A10 | *E. cloacae* | - | NA | 5.10⁶ |
| 38B2 | *C. freundii* | - | NA | 5.10⁶ |
| 38B7 | *K. pneumoniae* | - | NA | >10⁷ |
| 38B9 | *A. baumannii* | - | NA | 10⁶ |
| 38D6 | *E. cloacae* | - | NA | >10⁷ |
| 38J8 | *P. aeruginosa* | - | NA | 10⁶ |
| C105 | *A. baumannnii* | - | NA | >10⁷ |
| C111 | *P. aeruginosa* | - | NA | >10⁷ |
| C112 | *K. pneumoniae* | - | NA | 5.10⁶ |
| C115 | *K. pneumoniae* | - | NA | >10⁷ |
| C129 | *E. cloacae* | - | NA | >10⁷ |
| C130 | *K. pneumoniae* | - | NA | >10⁷ |
| C131 | *P. aeruginosa* | - | NA | >10⁷ |
| C132 | *K. pneumoniae* | - | NA | >10⁷ |
| C133 | *P. aeruginosa* | - | NA | 10⁶ |
| C136 | *C. freundii* | - | NA | >10⁷ |
| C140 | *E. cloacae* | - | NA | >10⁷ |
| C144 | *E. cloacae* | - | NA | >10⁷ |

| | | | | |
|---|---|---|---|---|
| ^{b}Underlined CFU counts are considered as negative results (cutoff values set at ≥ 1.10³ CFU/ml) ^{c}Positive culture after 36 hours | | | | |

### Results

The use of the EMB medium with the added components permits a visual identification of many enterobacterial species according to their color. Most of the colistin-resitant strains grew after 24 h. Naturally colistin-resistant *B. cepacia* and *S. maltophilia* grew after 36 h. The lowest limit of detection was below 1 x 10³ CFU/ml for all polymyxin-resistant strains whereas the limit of detection of all polymyxin-susceptible strains were above 1 x 10³ CFU/ml being ≥ 1 x 10⁶ CFU/ml. The sensitivity and specificity of the SUPERPOLYMYXIN medium for selecting polymyxin-resistant gram negatives was 100% in both cases. A further modification of the screening media could be addition of other chromogenic components for detecting specifically polymyxin-resistant *A*. *baumannii* and *P*. *aeruginosa.*

### References

Clinical and Laboratory Standards Institute. 2014. Performance standards for antimicrobial susceptibility testing; 24th Informational supplement. Document M100-S24. Clinical and Laboratory Standards Institute, Wayne, PA.
Falagas, M. E., P. I Rafailidis, and D. K. Matthaio. 2010. Resistance to polymyxins: Mechanisms, frequency and treatment options. Drug Resist. Update. 13:132-138.
IDSA Policy Paper. 2011. The bacterial challenge: time to react. 2009, Clin Infect Dis 52: S397-S428;
Gilligan, P. H., P.A Gage, L.M. Bradshaw, D.W Shidlow, and B.T. DeCicco. 1985. Isolation medium for the recovery of Pseudomonas cepacia from secretions of patients with cystic fibrosis. J Clin Microbiol 22:5-8.
Grasso, G.M, M.M. D'Errico F. Schioppa F. Romano, and D. Montanaro. 1988. Use of colistin and sorbitol for bettert isolation of Serratia marescens in clinical samples. Epidem Inf 101:315-320.
Hindler, J.A., and R. M. Humphries. 2013. Colistin MIC variability by method for contemporary clinical isolates of multidrug-resistant Gram-negative bacilli. J. Clin. Microbiol. 51:1678-1684.
Jayol, A., L. Poirel, A. Brink, M. V. Villegas, M. Yilmaz, and P. Nordmann. 2014 Resistance to colistin associated to a single amino acid change in protein PmrB among Klebsiella pneumoniae of worldwide origin. Antimicrob. Agents Chemother 58:4762-4766.
Kieffer, N., L. Poirel, P. Nordmann, J. Y. Madec, and M. Haenni. 2015. Emergence of colistin resistance in Klebsiella pneumoniae from veterinary medicine. J. Antimicrob. Chemother, in press.
Kumar, A., P. Ellis, Y. Arabi, D. Roberts, B. Light, J.E. Parillo, P. Dodek, G. Wodd, A. D. Simon, C. Peters, M. Ashan, D. Chateau, and Cooperative Antimicrobial Therapy of Septic shock database research group. 2009. Initiation of inappropriate antimicrobial therapy results in a fivefold reduction of survival in human septic shock. Chest 136: 1237-1248
Gales, A. C., R. N. Nones, and H. S. Sader. 2011. Contemporary activity of colistin and polymyxin B against a worldwide collection of Gram-negative pathogens: results from the Sentr Antimicrobial Surveillance Program (2006-09). J. Antimicrob. Chemother. 66:2070-2074.
Garnacho-Montero, J., T. Aldabo-Pallas, C. Garnacho-Montero, A. Cayuela, R. Jimenez, S. Barroso, and C. Ortiz-Leyba. 2006. Timing of adapted antibiotic therapy is a great determinant of outcome than are TNF and IL-10 polymorphisms in patients with sepsis. Critical Care 2006 10 R111.
Humphries, R. M. 2014 Susceptibility testing of polymyxins: where are we now? Pharmacotherapy 2014. doi:10.1002/phar.1505.
Hugh, R., and E. Leifson. 1953. The taxonomic significance of fermentative versus oxidative metabolism of carbohydrates by various gram negative bacteria. J Bacteriol 66: 24-26
Levine, M. 1918. Differentiation of B. coli and B. aerogenes on a simplified eosin-methylene blue agar. J. Infect Dis 23: 43-47.
Monaco, M., T. Giani, M. Raffone, F. Arena, A. Garcia-Fernandez, S. Pollini. 2014. Colistin resistance superimposed to endemic carbapenem-resistant Klebsiella pneumonia; a rapidly evolving problem in Italy, November 2013 to April 2014. Euro Surveill. 19. pii 20939.
Nation, R. L., J. Li, O. Cars, W. Couet, M. N. Dudley, K. S. Kaye, J. W. Mouton, D. L. Paterson, V. H. Tam, U. Theuretzbacher, B. Y. Tsuji, and J. Turnidge. 2014. Framework for optimisation of the clinical use of colistin and polymyxin B, the Prato polymyxins consensus. Lancet Infect. Dis., in press.
Olaitan, A. O., S. Morand and J. M. Rolain. 2014. Mechanisms of polymyxin resistance: acquired and intrinsic resistance in bacteria. Front. Microbiol. , in press
Park,Y.K., S.I. Jung, K. H. Park, H. S. Cheong, K . R. Peck, J. H. Song, and K. S. Ko. 2009. Independent emergence of colistin-resistnant Acinetobacter spp. isolates from Korea. Diagn. Microbiol. Infect. Dis., 64:43-51.
Poirel, L., A. Jayol, S. Bontron, M.-V. Villegas, M. Ozdemar, S. Türkoglu, and P. Nordmann. 2014. The mrgB gene as a key target for acquired resistance to colistin in Klebsiella pneumoniae. J. Antimicrob. Chemother., 70:75-80.
Nordmann, P., D. Girlich, and L. Poirel. 2012. Detection of carbapenemase producers in Enterobacteriaceae by use of a novel screening medium. J Clin Microbiol 50:2761-2766.
Nordmann, P., and L. Poirel. 2014. Emerging and important resistance in Gram negatives: epidemiology, theory and practice. Rev. Med. Suisse. 10:902-907.
Qureshi, Z. A., L. E. Hittle, J . A O'Hara, J. I. Rivera, A .Syed, R. K. Shields, A. W. Pasculle, R. K Ernst, and Y. Doi. 2015. Colistin-resistant Acinetobacter baumannii; beyond carbapenem resistance. Clin. Infect Dis. in press

## Claims

1. A culture medium comprising a polymyxin antibiotic, a daptomycin antibiotic and an antifungal agent.

2. The culture medium of claim 1, wherein said polymyxin antibiotic is selected from colistin, polymyxin B and mixtures thereof.

3. The culture medium of claim 1 or claim 2, wherein said polymyxin antibiotic is present at a concentration of 0.01 to 6 µg/ml.

4. The culture medium of any one of the preceding claims, wherein said polymyxin antibiotic is present at a concentration of 0.4 to 5 µg/ml, preferably 1 to 4 µg/ml, more preferably from > 2 to < 4 µg/ml.

5. The culture medium of any one of the preceding claims, wherein said daptomycin antibiotic is selected from daptomycin and analogues thereof, preferably it is daptomycin.

6. The culture medium of any one of the preceding claims, wherein said antifungal agent is amphotericin B.

7. The culture medium of any one of the preceding claims, wherein the concentration of said daptomycin antibiotic is 0.5 to 50 µg/ml, preferably 1 to 30 µg/ml, more preferably 5 to 15 µg/ml.

8. The culture medium of any one of the preceding claims, wherein the concentration of said antifungal agent is 0.1 to 50 µg/ml, preferably 0.5 to 25 µg/ml, more preferably 1 to 10 µg/ml.

9. The culture medium of any one of the preceding claims, further comprising eosine and/or methylene blue, preferably EMB agar.

10. A method for selecting gram-negative, aerobic bacteria that have an acquired resistance to polymyxins as well as bacteria that are naturally resistant to polymyxins, the method comprising the steps of:
- providing a culture medium as defined in any one of claims 1-9;
- inoculating said culture medium with a sample comprising bacteria;
- incubating said inoculated culture medium under conditions suitable for the growth bacteria exhibiting natural or acquired resistance to polymyxins;
- detecting colonies formed on said inoculated and cultivated medium, said colonies containing said bacteria that exhibit natural and/or acquired resistance to polymyxins.

11. The method of claim 10, wherein said bacteria that have an acquired resistance to polymyxins are bacteria belonging to taxa selected from the group of *Enterobacteriaceae, Pseudomonas aeruginosa* and *Acinetobacter baumannii.*

12. The method of claim 10, wherein said bacteria that have a natural resistance to polymyxins are bacteria belonging to taxa selected from the group of *Burkholderia*, *Proteus* and *Serratia.*

13. The method of any one of claims 10-12, wherein said incubation step is conducted for 24 hours to 48 hours, preferably 24 to 36 hours.

14. The method of any one of claims 10-13, wherein the detection of a bacteria that have an acquired resistance to polymyxins as well as bacteria that are naturally resistant to polymyxins, with the exception of *Burkholderia cepacia* and *Stenotrophomonas maltophilia,* is determined by detecting the occurrence of colonies within a culturing time of 24 to 36 hours.

15. The method of any one of claims 10-14, wherein the detection of naturally resistant *Burkholderia cepacia* and *Stenotrophomonas maltophilia* is determined by detecting the occurrence of colonies of a culturing time of 36 hours or more.
